# EUROPEAN PATENT SPECIFICATION

(11) **EP 2 461 815 B1**
(45) Date of publication and mention of the grant of the patent: **22.10.2014**
(21) Application number: 10744767.4
(22) Date of filing: 28.07.2010
(51) Int. Cl.: A61K 31/593, A61P 13/12

(54) **METHOD OF PREVENTING RENAL DISEASE AND TREATING SYMPTOMS THEREOF**
VERFAHREN ZUR VORBEUGUNG VON NIERENERKRANKUNGEN UND ZUR BEHANDLUNG VON SYMPTOMEN VON NIERENERKRANKUNGEN
METHODE POUR LA PRÉVENTION DE MALADIE RÉNALE ET LE TRAITEMENT DES SYMPTOMES DE MALADIE RÉNALE

(30) Priority: 03.08.2009 US 230861 P; 21.10.2009 US 253667 P
(43) Date of publication of application: 13.06.2012
(73) Proprietor: Wisconsin Alumni Research Foundation, Madison, WI 53705 (US)
(72) Inventor: CLAGETT-DAME, Margaret, Madison WI 53706 (US); PLUM, Lori, Arena WI 53503 (US); DELUCA, Hector, F., Deerfield WI 53531 (US); KIM, James, Wonkee, Daejon (KR)
(74) Representative: Duckworth, Timothy John
(86) International application number: PCT/US2010/043551
(87) International publication number: WO 2011/017165

(56) References cited:
- US-A1- 2002 028 830
- US-A1- 2005 187 201

## Description

### FIELD OF THE INVENTION

This invention relates to vitamin D compounds useful in preventing renal disease and treating symptoms thereof; more particularly to vitamin D compounds useful in preventing and treating symptoms of Lupus nephritis.

### BACKGROUND OF THE INVENTION

Renal disease has become an increasingly important health problem in virtually every country in the world including highly developed countries such as the United States. Presently there are 250,000 patients on renal dialysis who have lost almost complete use of their kidneys. There are approximately ten times that number of patients who have lost some degree of renal function due to renal disease and are progressing to complete renal failure. Renal failure is evidenced by a decreased glomeruli filtration rate (GFR) from a high value of 110 ml/minute to 30 ml/minute where dialysis is often initiated.

Many factors contribute to the development of renal disease. High blood pressure is one of the significant contributors, as is having Type I and Type II diabetes. Current treatments for renal failure are limited to hemodialysis, an extremely expensive procedure that currently is supported by federal governments because individuals cannot afford this procedure on their own. The annual cost of renal disease in the United States alone is over $42 billion. Accordingly, effective methods for preventing renal disease and treating symptoms thereof would not only provide a major health benefit but also a major economic benefit.

Subjects having renal disease are often treated with vitamin D compounds to reduce their secondary hyperparathyroidism that results as a consequence of renal disease. (See, for example, United States Published Patent Application numbers US 2005/0124591 and US 2006/0171983). Generally, however, vitamin D compounds are thought of as contributing to renal failure rather than playing a role in the treatment or prevention of renal disease.

US2002/0028830 discloses a method of treating SLE symptoms comprising administering an effective amount of vitamin D compound. 2 MD is not mentioned in the document.

US2005/0187201 discloses the use of 2MD in the treatment and prophylaxis of diseases where bone formation is desired, in particular osteoporosis.

Systemic Lupus Erythematosus (SLE) is a highly polymorphous, multigenic, non-organ-specific autoimmune disorder. It affects on average 15-50 people per 100,000 in a year. It mainly affects women (about 9 out of 10 cases), and certain ethnic populations seem predisposed to developing the disease, notably Afro-Caribbean, Afro-American and Spanish-American populations.

Although the origin of SLE is not known, it is a multifactor disease and different aetiological factors have been identified. As for genetic factors, susceptibility to the disease is clearly multigenic. Several genes have been shown to be implicated in genetic susceptibility, such as the DR2 and DR3 alleles of the HLA system. Other genes not related to the HLA system are also known to be involved. Moreover, environmental factors have also been identified as having a causal effect on SLE, such as ultraviolet radiation (photosensitive nature of lupus eruption) and sex hormones (women in periods of genital activity, role of and on pregnancy).

At the biological level, the characteristics of SLE include a generalized inflammatory syndrome during lupus flares, notably with a large amount of TNF-α secretion; haematological disorders and serological abnormalities, predominantly the existence of antinuclear antibodies (ANA) that can comprise anti-DNA, anti-histones, anti-nucleosomes, anti-Sm, anti-SSA or anti-SSB, and anti-ribonucleoprotein (RNP) antibodies. Patients also produce antibodies directed against figured elements of the blood or phospholipids; some of these auto-antibodies are able to participate in the formation of circulating immune complexes; and hypocomplementemia linked to the use of complement by immune complexes (related to severe kidney failure that improves during remissions), and/or a constitutional deficit of C2 or C4 (predisposing to SLE).

Lupus nephritis (LN) is the manifestation of SLE in the kidney. Lupus nephritis is a frequent manifestation in approximately 35-55% of subjects having SLE and is one of the main prognostic factors in diagnosing SLE. It can be detected by testing for haematuria (the presence of blood in the urine), leukocyturia (the presence of white blood cells in the urine), high blood pressure, or most commonly, proteinuria (elevated levels of protein in the urine). Detection of a nephropathy disease is a turning point that influences the prognosis of renal disease, because LN often can progress to chronic kidney failure within 5 to 10 years. In this case, patient survival can only be achieved with dialysis or a kidney transplant.

The central mechanism of LN development revolves around the formation and deposition of autoantibodies within kidney structures. One of the key pathways of LN pathogenesis is glomerular damage that triggers a cascade of local inflammatory and humoral responses resulting in global renal degradation.

The development of methods of preventing renal disease and treating symptoms thereof, including Lupus nephritis in particular, has been lacking. The currently approved treatments are limited in efficacy and have numerous and serious side effects. The active form of vitamin D₃ has proven to be a potent immunomodulatory agent, showing efficacy in treating various murine models of autoimmune diseases.

Therefore, a need exists for a vitamin D₃ compound that is effective in preventing renal disease and symptoms thereof in subjects at risk of developing renal disease as well as effective in treating (i.e., reducing the severity or slowing the progression of) symptoms of renal disease.

### SUMMARY OF THE INVENTION

We have now discovered that an analog of vitamin D is able to prevent renal disease and symptoms thereof.

In one embodiment, the present invention relates to a novel method of preventing renal disease by administering a therapeutically effective amount of a composition comprising 2-methylene-19-nor-20(S)-1,25-dihydroxyvitamin D₃ (2MD) or pharmaceutically acceptable salts thereof to a subject at risk of developing renal disease without inducing hypercalcemia in the subject.

In another embodiment, the present invention relates to methods of preventing symptoms of renal disease, including proteinuria, glomerular damage and splenomegaly, by administering a therapeutically effective amount of a composition comprising 2-methylene-19-nor-20(S)-1,25-dihydroxyvitamin D₃ (2MD) or pharmaceutically acceptable salts thereof to a subject at risk of developing symptoms of renal disease without inducing hypercalcemia in the subj ect.

In another embodiment, the present invention relates to a method of treating symptoms of renal disease by administering a therapeutically effective amount of a composition comprising 2-methylene-19-nor-20(S)-1,25-dihydroxyvitamin D₃ (2MD) or pharmaceutically acceptable salts thereof to a subject exhibiting symptoms of renal disease. In one embodiment the symptom of renal disease to be treated is proteinuria and/or splenomegaly.

In one embodiment, the 2-methylene-19-nor-20(S)-1,25-dihydroxyvitamin D₃ is formulated in an oral, topical, transdermal, parenteral, injection or infusion form in amounts ranging from 0.001 µg/day to about 5 µg/day per kg bodyweight.

Other features of the present invention will become apparent after review of the specification, claims and drawings.

### BRIEF DESCRIPTION OF DRAWINGS

Figure 1. (A) Molecular structure of 1α,25-dihydroxyvitamin D₃ (1,25(OH)₂D₃); (B) Molecular structure of 2-methylene-19-nor-(20S)-1α,25-(OH)₂D₃ (2MD).
Figure 2. Additional benefits of 2MD are increased survival, decreased PTH levels in the blood and improved bone mineral densities. The graphs depicting the serum PTH levels and total body BMD results represent the group averages and SEM (8-13 rats per group).
Figure 3. All markers of kidney failure are improved upon treatment with 2MD. Serum phosphorus and serum creatinine values represent averages and SEM of 8-13 uremic animals. Urine volume and protein measurements were obtained from 7-8 uremic animals six weeks after treatment initiation and are presented as averages and SEM.
Figure 4. All positive effects of 2MD are observed at dose levels that do not significantly raise serum calcium. Total serum calcium values shown are averages and SEM of 8-13 uremic animals.
Figure 5. Total urine volume and protein excretion are reduced in a model of type I diabetic nephropathy. Each bar represents the values obtained from 3-12 animals. The values shown are averages +/- SEM.

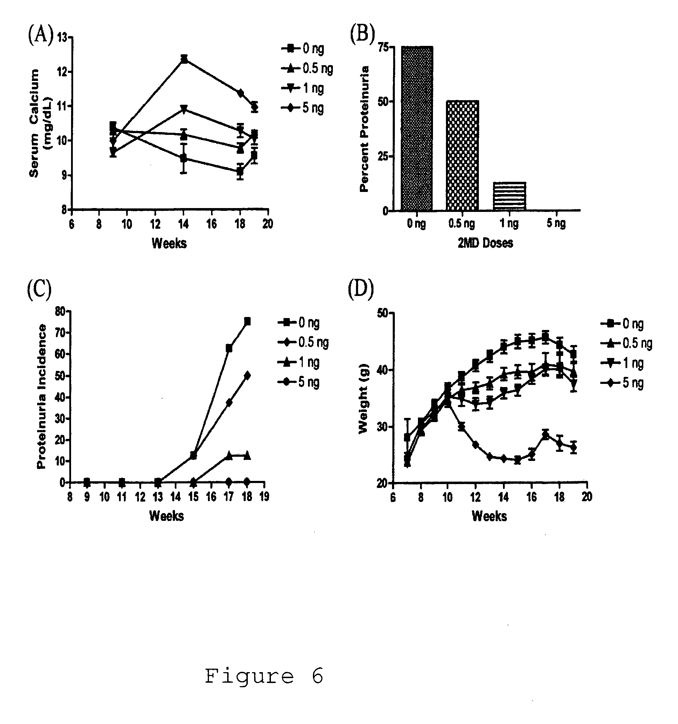

Figure 6. (A) Serum calcium levels are mildly elevated with 2MD treatment. Treatment was initiated at week 10, which is when mice first start showing signs of proteinuria. Study was terminated at week 19. (B) Terminal incidence of proteinuria. Proteinuria is completely prevented with 5 ng 2MD, and mice respond to lesser doses in dose dependent manner. (C) Mean onset of proteinuria is delayed with 1 ng 2MD treatment, and overall incidence is less for all dose groups. (D) Weight loss is most severe in the 5 ng 2MD group, while the stunted growth observed in the 0.5 ng and 1 ng groups is very mild. (n=8)

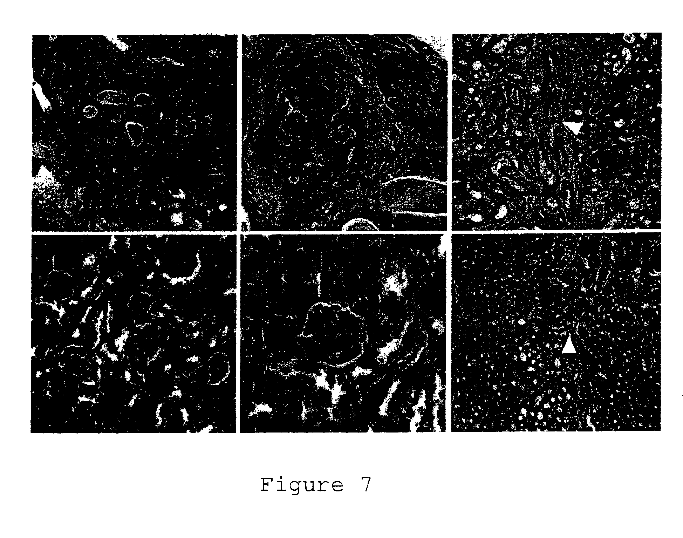

Figure 7. (A) and (D) are 100x micrographs of control and 2MD treated kidneys, respectively. 'G' denotes glomeruli. (B) and (E) are 400x micrographs of (A) and (D), respectively. In (B), there is marked glomerular sclerosis, and fibrosis (blue staining) is visible as well. Protein casts, indicative of proteinuria, are visible in the lower right corner of (B). Although interstitial fibrosis was present in both control (C) and treated (F) samples, the extent and frequency was much less in the 2MD treated samples. White arrowheads point out the blue staining of fibrotic areas. All sections were trichrome stained.

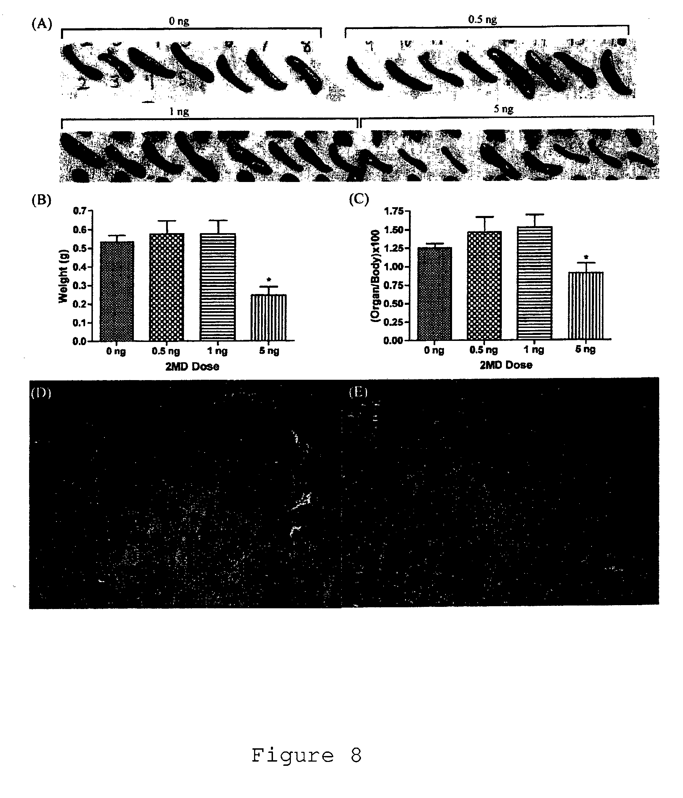

Figure 8. (A) Photograph of spleens. Brackets indicate dose of 2MD administered. Note the significantly smaller spleens present only in the 5 ng 2MD group. (B) Non-normalized spleen weights. Reflecting the visual observation in (A), spleen weights are only significantly decreased in the 5 ng 2MD group. (C) Spleen weights normalized to body weight. The pattern is almost identical to (B). (n=8) *p<0.05 (D) and (E) are 40x micrographs of H&E stained control and 2MD treated group spleens, respectively. (D) shows disorganized white pulp structure, while white pulp in (E) retains some of the sheath structure that is normal in non-diseased spleens.

### DETAILED DESCRIPTION OF THE EXEMPLARY EMBODIMENTS

### I. IN GENERAL

In the specification and in the claims, the terms "including" and "comprising" are open-ended terms and should be interpreted to mean "including, but not limited to.... " These terms encompass the more restrictive terms "consisting essentially of" and "consisting of."

As used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", "characterized by" and "having" can be used interchangeably.

Unless defined otherwise, all technical and scientific terms used herein have the same meanings as commonly understood by one of ordinary skill in the art to which this invention belongs. All publications and patents are mentioned herein with the purpose of describing and disclosing the chemicals, instruments, statistical analyses and methodologies which are reported in the publications which might be used in connection with the invention. All references cited in this specification are to be taken as indicative of the level of skill in the art.

### II. THE INVENTION

Previously, this laboratory demonstrated that 300 ng per day of 1α,25-dihyroxyvitamin D₃ (1,25(OH)₂D₃) administered through the diet can effectively prevent renal disease and renal failure by reducing the symptoms of renal disease. James Wonkee Kim. Effects of 1α,25-dihydroxyvitamin D3 on the MRL/MpJ-Fas/lpr model of systemic lupus erythematosus. Ph.D. These, University of Wisconsin-Madison (2009). For instance, the inventors have previously shown that administering 1α,25-dihyroxyvitamin D₃ (1,25(OH)₂D₃) completely prevents proteinuria in the MRL/MpJ-Fas^{lpr} (MRL/lpr) mouse model of systemic lupus erythematosus (SLE). James Kid, Id. However, severe hypercalcemia always accompanied this treatment. Hypercalcemia (increased levels of calcium in the blood) can result in serious physical problems, including death. Specifically, an increase in calcium of approximately 2 mg/100 ml is considered mild hypercalcemia and is not considered a problem. However, an increase in calcium levels of more than 2mg/100ml is considered sever hypercalcemia and can cause calcification of the kidney, heart, and aorta. Clearly, the use of this compound is not optimal because of the resultant hypercalcemia. We have therefore continued to search for compounds that will prevent renal disease and symptoms thereof without inducing hypercalcemia. Compounds that will treat (i.e., reduce the severity or slow the progression of) symptoms of renal disease without inducing hypercalcemia are also desired.

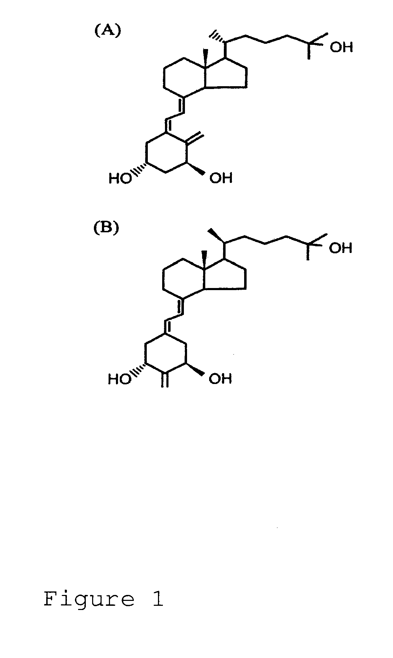

2-methylene-19-nor-(20S)-1α,25-(OH)₂D₃ (2MD) is an analog of 1,25(OH)₂D₃ (Figure 1) which has been shown to have increased *in vivo* potency toward bone but not on intestinal calcium absorption. The overall synthesis of 2MD is illustrated and described more completely in U.S. Pat. No. 5,843,928, issued Dec. 1, 1998, and entitled "2-Alkylidene-19-Nor-Vitamin D Compounds". The biological activity of 2MD is reported in U.S. Patent Application Ser. No. 09/616,164, filed Jul. 14, 2000.

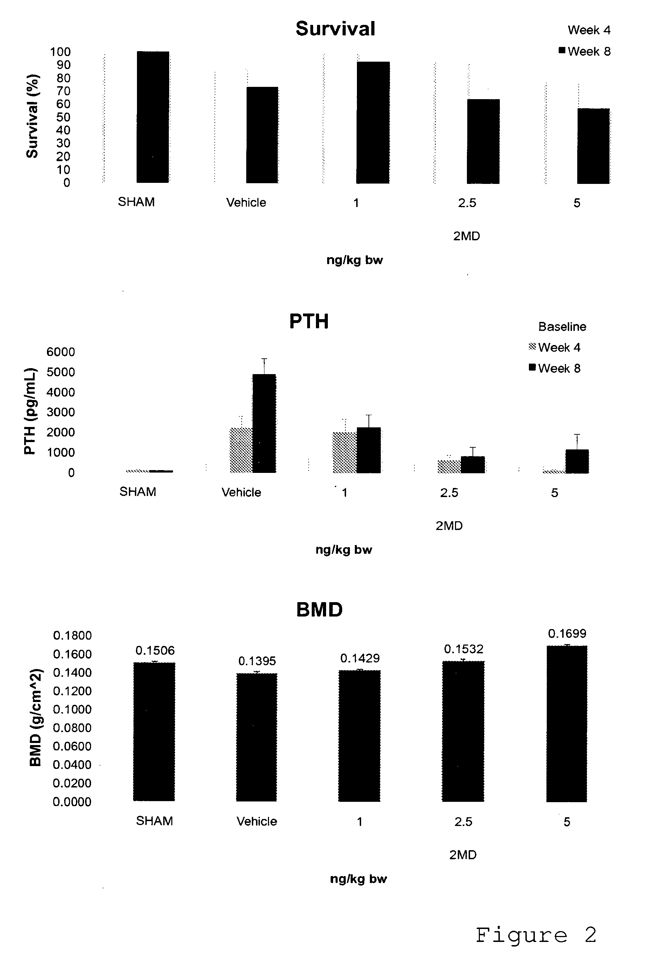

The present invention therefore provides that 2MD can prevent renal disease and various symptoms thereof (including proteinuria, glomerular damage and splenomegaly) without causing severe hypercalcemia, while also exhibiting increased survival, decreased PTH levels in the blood and improved bone mineral densities (Figure 2).

The present invention also shows that 2MD can treat (i.e., reduce the severity and slow progression of) various symptoms of renal disease without causing severe hypercalcemia, such symptoms including proteinuria and splenomegaly.

By "hypercalcemia" we mean elevated calcium levels in the blood of more than 2mg/100ml. In a normal subject, calcium levels are approximately 9-10.5 mg/dL or 2.2-2.6 mmol/L. In cases of severe hypercalcemia (i.e., calcium levels above 15-16 mg/dL or 3.75-4 mmol/l) coma and cardiac arrest can develop.

In one embodiment, the present invention therefore provides novel methods of preventing renal disease in a subject at risk of developing renal disease by administering to the subject a therapeutically effective amount of 2-methylene-19-nor-(20S)-1α,25-(OH)₂D₃ (2MD) or pharmaceutically acceptable salts thereof without inducing hypercalcemia in the subject, where 2MD has the structure (I):

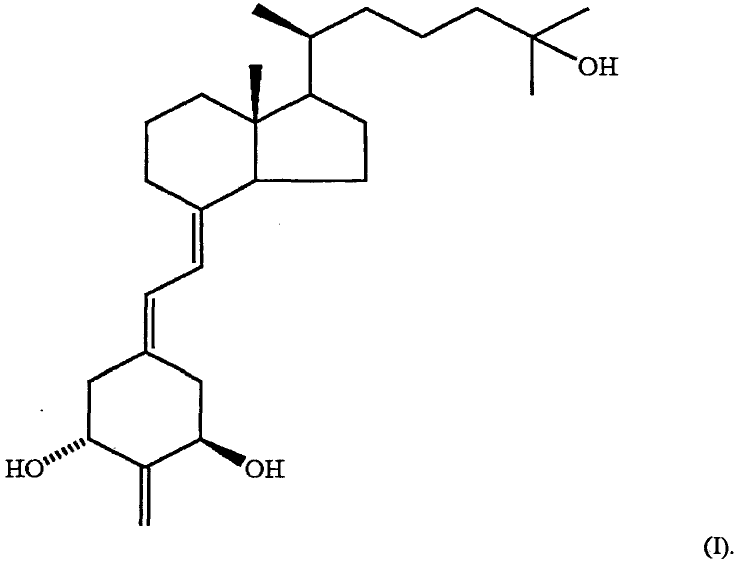

(The double bond coming off the ring denotes a =(CH₂) and the structure is technically correct without the (CH₂) actually shown.)

By "preventing" we mean a forestalling of a clinical symptom indicative of renal disease and/or lupus nephritis. Such forestalling includes, for example, the maintenance of normal kidney functions in a subject at risk of developing renal disease prior to the development of overt symptoms of renal disease including but not limited to proteinuria, glomerular damage or splenomegaly. Therefore, the term "preventing" includes the prophylactic treatment of subjects to guard them from the occurrence of renal disease. Preventing renal disease in a subject is also intended to include inhibiting or arresting the development of renal disease. Inhibiting or arresting the development of renal disease includes, for example, inhibiting or arresting the occurrence of proteinuria, glomerular damage, splenomegaly and other symptoms of renal disease. Inhibiting or arresting the development of renal disease also includes, for example, inhibiting or arresting the progression of one or more pathological conditions or chronic complications associated with renal disease.

By "renal disease" we mean a condition exhibiting impaired kidney function in a subject who is not on dialysis or a patient with chronic kidney disease (CKD) at stages 2 or 3, such as, for instance, acute kidney failure, acute nephritic syndrome, analgesic nephropathy, atheroembolic renal disease, chronic kidney failure, chronic nephritis, congenital nephrotic syndrome, goodpasture syndrome, interstitial nephritis, kidney cancer, kidney damage, kidney infection, kidney injury, kidney stones, membranoproliferative GNI, membranoproliferative GNII, membranous nephropathy, minimal change disease, necrotizing glomerulonephritis, nephroblastoma, nephrocalcinosis, nephrogenic diabetes insipidus, nephropathy - IgA, nephrosis nephrotic syndrome, polycystic kidney disease, post-streptococcal GN, reflux nephropathy, renal artery embolism, renal artery stenosis, renal disorders, renal papillary necrosis, renal tubular acidosis type I, renal tubular acidosis type II, renal underperfusion, renal vein thrombosis.

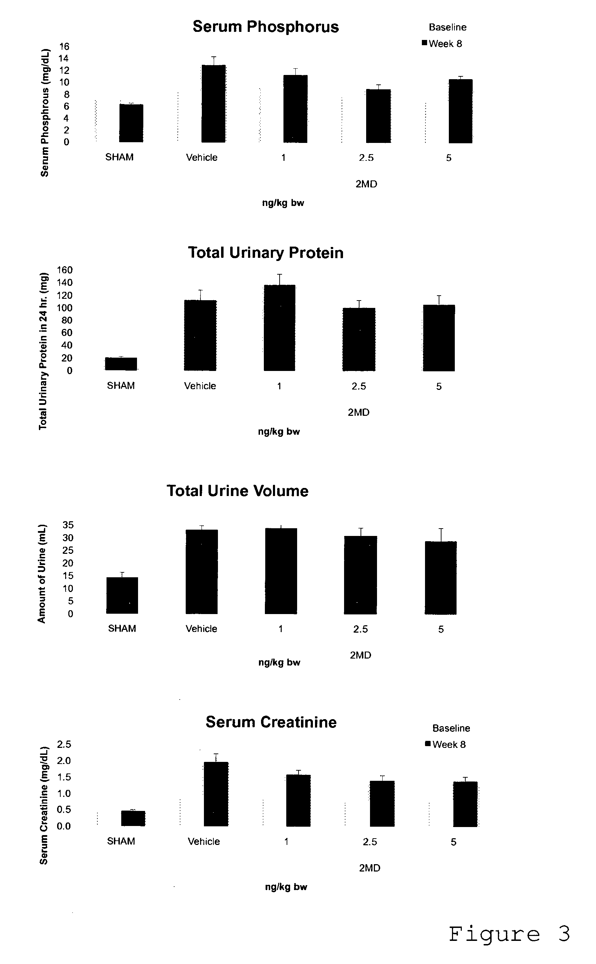

We do not mean to include patients with established kidney failure (e.g. a glomerular filtration rate (GFR) of <15 mL/min/1.73 m² or permanent renal replacement therapy (RRT)). By "renal disease" we also mean a subject who has had kidney damage for ≥ 3 months, as defined by structural or functional abnormalities of the kidney, with or without decreased GFR, manifested by either pathological abnormalities or markers of kidney damage, including abnormalities in the composition of the blood or urine, or abnormalities in imaging tests (Figure 3). Markers of kidney damage include proteinuria of > 300 µg/day as measured by 24-HR excretion method (See Table 15, Am. J. of Kidney Diseases, v. 39, no. 2, Suppl. 1 (Feb. 2002), pp. 546-575, incorporated by reference). This definition does not include patients on dialysis.

By "stage 2 CKD" we mean a mild reduction in GFR (60-89 mL/min/1.73 m²) with kidney damage. Kidney damage is defined as pathologic abnormalities or markers of damage, including abnormalities in blood or urine test or imaging studies. By "stage 3 CKD" we mean a moderate reduction in GFR (30-59 mL/min/1.73 m²). British guidelines distinguish between stage 3A (GFR 45-59) and stage 3B (GFR 30-44) for purposes of screening and referral. For more information about both stages see Am. J. of Kidney Disease, v. 39, No. 2, Suppl. 1, Feb 2002, incorporated by reference.

In one embodiment the renal disease is lupus nephritis. By "lupus nephritis" we mean the chronic kidney disease characterized by glomerular immune complex (IC) deposition, glomerular and tubular inflammation and interstitial infiltration of leukocytes. Lupus nephritis can create serious complications including toxicity from renal failure, hypertension and cardiovascular failure from renal insufficiency, and an increased susceptibility to infections. A subject having lupus nephritis exhibits at least four of the following symptoms: a facial rash in butterfly configuration; discoid lupus eruption; photosensitivity; mouth or nasopharyngeal ulcers; non-erosive polyarthritis, pleuritis or pericarditis; proteinuria >0.5 g/24 h or cylindruria; seizures or psychosis; haemolytic anemia or leucopenia <4,000/mm³ or lymphopenia <1,500/mm³ or thrombocytopenia <100,000/mm³; immunologic abnormalities including the presence of LE cells (Hargraves cells) or anti-native DNA antibodies (Abs) or anti-Sm antibodies or false positive syphilis result; or the presence of a large number of antinuclear Abs.

By "subject" we mean mammals and non-mammals. "Mammals" means any member of the class Mammalia including, but not limited to, humans, non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

By "at risk for developing renal disease" we mean any subject having renal disease, such as SLE, or at risk of having renal disease, such as SLE.

By "administering" we mean any means for introducing a compound into the body, preferably into the systemic circulation, as described in more detail below. Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

By "therapeutically effective" we mean an amount of a compound that, when administered to a subject for treating a disease, is sufficient to effect such treatment for the disease. The "therapeutically effective amount" will vary depending on the compound, the disease state being treated, the severity or the disease treated, the age and relative health of the subject, the route and form of administration, the judgment of the attending medical or veterinary practitioner, and other factors. We dosed MRL/lpr mice with several dose levels of 2MD that would not cause significant hypercalcemia. We found that 5 ng of 2MD per mouse per day is sufficient to prevent proteinuria without dramatically increasing the subject's serum calcium levels. Furthermore, 1 ng of 2MD showed over a 60% reduction in proteinuria incidence while maintaining serum calcium levels within the physiologically normal range.

In one embodiment, the therapeutically effective amount ranges from between about 0.001-5 µg/day. In an alternative embodiment, the therapeutically effective amount ranges from between about 0.025-0.3 µg/day. (See US Patent 5,843,928, which discloses suitable methods of administration). One would monitor the progression of renal disease by tracking symptoms, such as total urinary protein excretion, creatinine, GFR and phosphorus. One would expect these symptoms to slow in progression.

In another embodiment, the present invention provides a novel method of treating symptoms of renal disease. The method comprises administering to a subject exhibiting symptoms of renal disease a therapeutically effective amount of 2MD without inducing hypercalcemia in the subject. In one embodiment the symptom to be treated is proteinuria, and/or splenomegaly.

By "treating" we mean an amelioration of a clinical symptom indicative of renal disease. Amelioration of a clinical symptom includes, for example, reducing the severity or slowing the progression of a symptom of renal disease. For instance, limiting proteinuria by lowering the amount of serum protein released from the kidneys in response to treatment with 2MD, or reducing the size of the spleen of a subject having splenomegaly. Specifically, we mean reducing the amount of protein in the urine by at least about 20%. In one embodiment, the amount of protein in the subject's urine is reduced by about 20-40% or about 35-50%. We also mean reducing the size of the spleen by at least about 20%. In one embodiment, the subject's spleen size is reduced by about 20-40% or about 35-50%. Other pathological conditions, chronic complications or phenotypic manifestations of renal disease are known to those skilled in the art and can similarly be used as a measure of treating renal disease so long as there is a reduction in the severity of the condition, complication or manifestation associated with the disease.

Specifically, in one embodiment, the present invention treats or prevents proteinuria in a subject. By "proteinuria" we mean the presence of an excess of serum proteins in the urine. The protein in the urine often causes the urine to become foamy. Since serum proteins are readily reabsorbed from urine, the presence of excess protein indicates either an insufficiency of absorption or impaired filtration.

In another embodiment, the present invention prevents additional glomerular damage in a subject. By "glomerular damage" we mean damage to the glomerulus, the tiny clusters of looping blood vessels inside a subject's kidneys. Blood enters the kidneys through arteries that branch inside the kidneys into the glomerulus, which comes from the Greek word meaning filter. There are approximately 1 million glomeruli, or filters, in each kidney. The glomerulus is attached to the opening of a small fluid-collecting tubules. Blood is filtered in the glomerulus, and extra water and wastes pass into the tubules and become urine. Eventually, the urine drains from the kidneys into the bladder through the ureters. When damaged, the glomeruli let protein and sometimes red blood cells leak into the urine. Glomerular damage can also interfere with the clearance of waste products by the kidney, so they begin to build up in the blood. Furthermore, loss of blood proteins like albumin in the urine can result in a fall in their level in the bloodstream. In normal blood, albumin acts like a sponge, drawing extra fluid from the body into the bloodstream, where it remains until the kidneys remove it. But when albumin leaks into the urine, the blood loses its capacity to absorb extra fluid from the body. Fluid can accumulate outside the circulatory system in the face, hands, feet, or ankles and cause swelling. Glomerular damage can include proteinuria (large amounts of protein in the urine); hematuria (blood in the urine); reduced glomerular filtration rate (inefficient filtering of wastes from the blood); hypoproteinemia (low blood protein) and edema (swelling in parts of the body).

In another embodiment, the present invention treats or prevents splenomegaly in a subject. By "splenomegaly" we mean an enlargement of the spleen, which usually lies in the left upper quadrant of the human abdomen. It is usually associated with increased workload (such as in hemolytic anemias), which suggests that it is a response to hyperfunction. It is associated with any disease process that involves abnormal red blood cells being destroyed in the spleen. Splenomegaly is defined as spleen size > 12 cm. Moderate splenomegaly is defined as a spleen size between 11-20 cm and severe splenomegaly is defined as a spleen size greater than 20 cm. Treatment usually involves a splenectomy, wherein the spleen is removed. However, this leaves subjects vulnerable to an increased risk for infectious diseases.

By "therapeutically effective" we mean an amount of a compound that, when administered to a subject for treating a disease, is sufficient to effect such treatment for the disease. The "therapeutically effective amount" will vary depending on the compound, the disease state being treated, the severity or the disease treated, the age and relative health of the subject, the route and form of administration, the judgment of the attending medical or veterinary practitioner, and other factors. In one embodiment, the therapeutically effective amount ranges from between about 0.001-5 µg/day per kg bodyweight. In an alternative embodiment, the therapeutically effective amount ranges from between about 0.025-0.3 µg/day. One would monitor the progression of renal disease by tracking symptoms, such as total urinary protein excretion, creatinine, GFR and phosphorus. One would expect these symptoms to slow in progression.

In one embodiment of the invention, one would combine 2MD treatment with angiotensin receptor blockers (ARBs) or angiotensin converting enzyme inhibitors (ACE inhibitors) used to treat hypertension.

Effective compound formulations are described in U.S. Patent 5,843,928 and include pharmaceutical applications as a solution in innocuous solvents, or as an emulsion, suspension or dispersion in suitable solvents or carriers, or as pills, tablets, capsules combined with solid carriers. Other formulations may also include other pharmaceutically acceptable and nontoxic excipients such as stabilizers, anti-oxidants, binders, coloring agents or emulsifying or taste-modifying agents and extended release formulations.

### Dosage.

In one embodiment, the 2MD compound is the active pharmaceutical ingredient (API) of this invention. The API may be formulated in an oral pharmaceutical dosage form as a solution in innocuous solvents, emulsion, suspension or dispersion in suitable solvents or carriers. The API may also be formulated in various oral dosage forms, such as pills, tablets or capsules using suitable pharmaceutical solid carriers. Such pharmaceutical formulations may also contain other pharmaceutically suitable USP-approved inactive ingredients, excipients, such as stabilizers, anti-oxidants, binders, coloring agents, emulsifiers, and/or taste-modifying agents, which are referred to as USP approved inactive pharmaceutical ingredients.

The API may be administered orally, topically, parenterally or transdermally or by inhalation. The compound may be administered by injection or intravenous infusion using suitable sterile solutions. Topical dosage forms may be creams, ointments, patches, or similar vehicles suitable for transdermal and topical dosage forms.

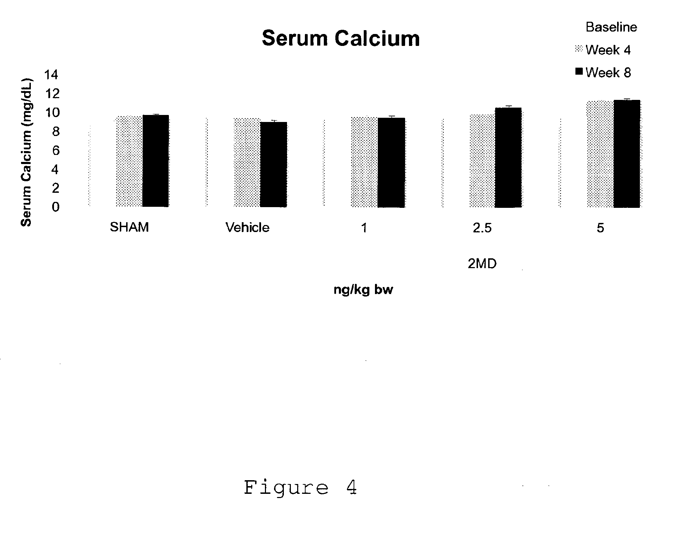

Doses in the range of 0.1 µg to 10 µg per day per kg bodyweight of the API may be used for the prevention or treatment of renal failure/proteinuria. All positive effects of 2MD are observed at dose levels that do not significantly raise serum calcium (Figure 4). Such doses and dosing regimens may be adjusted to accommodate disease severity or progression, patient predisposition/at-risk/susceptible-to and other known criteria.

The pharmaceutically suitable oral carrier systems (also referred to as drug delivery systems, which are modem technology, distributed with or as a part of a drug product that allows for the uniform release or targeting of drugs to the body) preferably include FDA-approved and/or USP-approved inactive ingredients. Under 21 CFR 210.3(b)(8), an inactive ingredient is any component of a drug product other than the active ingredient. According to 21 CFR 210.3(b)(7), an active ingredient is any component of a drug product intended to furnish pharmacological activity or other direct effect in the diagnosis, cure, mitigation, treatment, or prevention of disease, or to affect the structure or any function of the body of humans or other animals. Active ingredients include those components of the product that may undergo chemical change during the manufacture of the drug product and be present in the drug product in a modified form intended to furnish the specified activity or effect. As used herein, a kit (also referred to as a dosage form) is a packaged collection of related material.

### Administration.

As used herein, the oral dosage form includes capsules, a solid oral dosage form consisting of a shell and a filling, whereby the shell is composed of a single sealed enclosure, or two halves that fit together and which are sometimes sealed with a band, and whereby capsule shells may be made from gelatin, starch, or cellulose, or other suitable materials, may be soft or hard, and are filled with solid or liquid ingredients that can be poured or squeezed. The oral dosage form may also be a capsule or coated pellets, in which the drug is enclosed within either a hard or soft soluble container or "shell" made from a suitable form of gelatin. The drug itself may be in the form of granules to which varying amounts of coating have been applied or in a capsule coated extended release, in which the drug is enclosed within either a hard or soft soluble container or "shell" made from a suitable form of gelatin. Additionally, the capsule may be covered in a designated coating which releases a drug or drugs in such a manner to allow at least a reduction in dosing frequency as compared to that drug or drugs presented as a conventional dosage form.

The oral dosage form may further be a capsule delayed release, in which the drug is enclosed within either a hard or soft soluble container made from a suitable form of gelatin, and which releases a drug (or drugs) at a time other than promptly after administration, whereby enteric-coated articles are delayed release dosage forms. Capsule delayed release pellets, in which the drug is enclosed within either a hard or soft soluble container or "shell" made from a are also useful. In these cases, the drug itself is in the form of granules to which enteric coating has been applied, thus delaying release of the drug until its passage into the intestines. Capsule extended release and capsule film-coated extended release are also useful.

Additionally, the capsule is covered in a designated film coating, and which releases a drug or drugs in such a manner to allow at least a reduction in dosing frequency as compared to that drug or drugs presented as a conventional dosage form), capsule gelatin coated (a solid dosage form in which the drug is enclosed within either a hard or soft soluble container made from a suitable form of gelatin; through a banding process, the capsule is coated with additional layers of gelatin so as to form a complete seal), capsule liquid filled (a solid dosage form in which the drug is enclosed within a soluble, gelatin shell which is plasticized by the addition of a polyol, such as sorbitol or glycerin, and is therefore of a somewhat thicker consistency than that of a hard shell capsule.

Typically, the active ingredients are dissolved or suspended in a liquid vehicle), granule (a small particle or grain), pellet (a small sterile solid mass consisting of a highly purified drug, with or without excipients, made by the formation of granules, or by compression and molding), pellets coated extended release (a solid dosage form in which the drug itself is in the form of granules to which varying amounts of coating have been applied, and which releases a drug or drugs in such a manner to allow a reduction in dosing frequency as compared to that drug or drugs presented as a conventional dosage form).

Other forms include pills (a small, round solid dosage form containing a medicinal agent intended for oral administration), powder (an intimate mixture of dry, finely divided drugs and/or chemicals that may be intended for internal or external use), elixir (a clear, pleasantly flavored, sweetened hydroalcoholic liquid containing dissolved medicinal agents; it is intended for oral use), chewing gum (a sweetened and flavored insoluble plastic material of various shapes which when chewed, releases a drug substance into the oral cavity), syrup (an oral solution containing high concentrations of sucrose or other sugars; the term has also been used to include any other liquid dosage form prepared in a sweet and viscid vehicle, including oral suspensions), tablet (a solid dosage form containing medicinal substances with or without suitable diluents), tablet chewable (a solid dosage form containing medicinal substances with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained medicament available over an extended period of time following ingestion.

In other forms, a tablet for solution, tablet for suspension, tablet multilayer, tablet multilayer extended release may be provided, where the tablet is formulated in such manner as to allow at least a reduction in dosing frequency as compared to that drug presented as a conventional dosage form. A tablet orally disintegrating, tablet orally disintegrating delayed release, tablet soluble, tablet sugar coated, osmotic, and the like are also suitable.

The oral dosage form composition contains an active pharmaceutical ingredient and one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer, whereby inactive ingredients can also be considered active ingredients under certain circumstances, according to the definition of an active ingredient given in 21 CFR 210.3(b)(7). Alcohol is a good example of an ingredient that may be considered either active or inactive depending on the product formulation.

As used herein, the injectable and infusion dosage forms include, but are not limited to, a liposomal injectable, which either consists of or forms liposomes (a lipid bilayer vesicle usually composed of phospholipids which is used to encapsulate an active drug substance). An injection, which includes a sterile preparation intended for parenteral use; five distinct classes of injections exist as defined by the USP, is also suitable. An emulsion injection, which includes an emulsion consisting of a sterile, pyrogen-free preparation intended to be administered parenterally or a lipid complex injection are also suitable.

Other forms include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate an active drug substance, either within a lipid bilayer or in an aqueous space) to be formed upon reconstitution; a powder lyophilized for solution injection, which is a dosage form intended for the solution prepared by lyophilization ("freeze drying"), a process which involves the removal of water from products in the frozen state at extremely low pressures.

This is intended for subsequent addition of liquid to create a solution that conforms in all respects to the requirements for injections; a powder lyophilized for suspension injection being a liquid preparation, intended for parenteral use that contains solids suspended in a suitable fluid medium and conforms in all respects to the requirements for Sterile Suspensions; the medicinal agents intended for the suspension are prepared by lyophilization ("freeze drying"), a process which involves the removal of water from products in the frozen state at extremely low pressures; a solution injection being a liquid preparation containing one or more drug substances dissolved in a suitable solvent or mixture of mutually miscible solvents that is suitable for injection; a solution concentrate injection being a sterile preparation for parenteral use which, upon the addition of suitable solvents, yields a solution conforming in all respects to the requirements for injections.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate an active drug substance, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the active ingredient which is preferably isotonic with the blood of the recipient.

As used herein, inhalation dosage forms include, but are not limited to, aerosol being a product that is packaged under pressure and contains therapeutically active ingredients that are released upon activation of an appropriate valve system intended for topical application to the skin as well as local application into the nose (nasal aerosols), mouth (lingual and sublingual aerosols), or lungs (inhalation aerosols); foam aerosol being a dosage form containing one or more active ingredients, surfactants, aqueous or nonaqueous liquids, and the propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged; metered aerosol being a pressurized dosage form consisting of metered dose valves which allow for the delivery of a uniform quantity of spray upon each activation; powder aerosol being a product that is packaged under pressure and contains therapeutically active ingredients, in the form of a powder, that are released upon activation of an appropriate valve system; and, aerosol spray being an aerosol product which utilizes a compressed gas as the propellant to provide the force necessary to expel the product as a wet spray and being applicable to solutions of medicinal agents in aqueous solvents.

As used herein, transdermal dosage form includes, but is not limited to, a patch being a drug delivery system that often contains an adhesive backing that is usually applied to an external site on the body, whereby the ingredients either passively diffuse from, or are actively transported from, some portion of the patch, and whereby depending upon the patch, the ingredients are either delivered to the outer surface of the body or into the body; and, other various types of transdermal patches such as matrix, reservoir and others known in the art.

As used herein, the topical dosage form includes various dosage forms known in the art such as lotions (an emulsion, liquid dosage form, whereby this dosage form is generally for external application to the skin), lotion augmented (a lotion dosage form that enhances drug delivery, whereby augmentation does not refer to the strength of the drug in the dosage form), gels (a semisolid dosage form that contains a gelling agent to provide stiffness to a solution or a colloidal dispersion, whereby the gel may contain suspended particles) and ointments (a semisolid dosage form, usually containing <20% water and volatiles and >50% hydrocarbons, waxes, or polyols as the vehicle, whereby this dosage form is generally for external application to the skin or mucous membranes).

Ointment augmented (an ointment dosage form that enhances drug delivery, whereby augmentation does not refer to the strength of the drug in the dosage form), creams (an emulsion, semisolid dosage form, usually containing > 20% water and volatiles and/or < 50% hydrocarbons, waxes, or polyols may also be used as the vehicle, whereby this dosage form is generally for external application to the skin or mucous membranes. Cream augmented (a cream dosage form that enhances drug delivery, whereby augmentation does not refer to the strength of the drug in the dosage form), emulsions (a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable, e.g. cream, lotion, ointment), suspensions (a liquid dosage form that contains solid particles dispersed in a liquid vehicle), suspension extended release, pastes (a semisolid dosage form, containing a large proportion, 20 - 50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders are also suitable.

Shampoos (a lotion dosage form which has a soap or detergent that is usually used to clean the hair and scalp) are often used as a vehicle for dermatologic agents. For instance, shampoo suspensions (a liquid soap or detergent containing one or more solid, insoluble substances dispersed in a liquid vehicle that is used to clean the hair and scalp and is often used as a vehicle for dermatologic agents) are often used. Aerosol foams (i.e., a dosage form containing one or more active ingredients, surfactants, aqueous or nonaqueous liquids, and the propellants; if the propellant is in the internal discontinuous phase, i.e., of the oil-in-water type, a stable foam is discharged, and if the propellant is in the external continuous phase, i.e., of the water-in-oil type, a spray or a quick-breaking foam is discharged), sprays (a liquid minutely divided as by a jet of air or steam), metered spray (a non-pressurized dosage form consisting of valves which allow the dispensing of a specified quantity of spray upon each activation), and suspension spray (a liquid preparation containing solid particles dispersed in a liquid vehicle and in the form of coarse droplets or as finely divided solids to be applied locally, most usually to the nasal-pharyngeal tract, or topically to the skin) are also suitable.

Jellies (a class of gels, which are semisolid systems that consist of suspensions made up of either small inorganic particles or large organic molecules interpenetrated by a liquid--in which the structural coherent matrix contains a high portion of liquid, usually water) and films (a thin layer or coating), including film extended release (a drug delivery system in the form of a film that releases the drug over an extended period in such a way as to maintain constant drug levels in the blood or target tissue) and film soluble (a thin layer or coating which is susceptible to being dissolved when in contact with a liquid) are also suitable.

Sponges (a porous, interlacing, absorbent material that contains a drug, whereby it is typically used for applying or introducing medication, or for cleansing, and whereby a sponge usually retains its shape), swabs (a small piece of relatively flat absorbent material that contains a drug, whereby a swab may also be attached to one end of a small stick, and whereby a swab is typically used for applying medication or for cleansing).

Patches (a drug delivery system that often contains an adhesive backing that is usually applied to an external site on the body, whereby its ingredients either passively diffuse from, or are actively transported from, some portion of the patch, whereby depending upon the patch, the ingredients are either delivered to the outer surface of the body or into the body, and whereby a patch is sometimes synonymous with the terms 'extended release film' and 'system'), patch extended release (a drug delivery system in the form of a patch that releases the drug in such a manner that a reduction in dosing frequency compared to that drug presented as a conventional dosage form, e.g., a solution or a prompt drug-releasing, conventional solid dosage form), patch extended release electronically controlled (a drug delivery system in the form of a patch which is controlled by an electric current that releases the drug in such a manner that a reduction in dosing frequency compared to that drug presented as a conventional dosage form, e.g., a solution or a prompt drug-releasing, conventional solid dosage form), and the like. The various topical dosage forms may also be formulated as immediate release, controlled release, sustained release, or the like.

The topical dosage form composition contains an active pharmaceutical ingredient and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer, whereby inactive ingredients can also be considered active ingredients under certain circumstances, according to the definition of an active ingredient given in 21 CFR 210.3(b)(7). Alcohol is a good example of an ingredient that may be considered either active or inactive depending on the product formulation.

While multiple embodiments are disclosed, still other embodiments of the present invention will become apparent to those skilled in the art from the following detailed description.

The detailed description of the methods of the present invention are to be regarded as illustrative in nature.

### EXAMPLES

The following examples are, of course, offered for illustrative purposes only.

In short, the examples below illustrate that 2MD, an analog of 1,25(OH)₂D₃ originally thought to be important in prevention and treatment of osteoporosis, is also important in slowing the progression of renal disease and treating symptoms thereof. A study conducted in rats in which 5/6ths of their kidneys were surgically removed, showed that daily oral 2MD treatment results in lower levels of phosphorus, creatinine, urinary volume and protein excretion, all indicators of kidney failure, compared to vehicle control animals. Furthermore this compound results in increased overall survival, increased bone mineral density, and lower PTH levels at dose levels that do not raise serum calcium. A second animal model of type I diabetic nephropathy confirms the results obtained in the uremic rat model. Mice provided 2MD in the diet show marked improvement in total urinary protein excretion as well an overall urine volume output.

### Example 1: Methods and Materials.

Mice. All mice used in this study were female MRL/MpJ-Fas^{lpr}/J mice either purchased directly or originally purchased from The Jackson Laboratory and bred in house. All mice involved in each study were age matched to the week of birth. All animal handling was performed according to protocol A1205 approved by the University of Wisconsin - Madison Institutional Animal Care and Use Committee. From weaning, female mice were placed on purified diet containing 0.47% calcium without 2MD until treatment initiation. The purified powder diet was prepared in agar form and administered 3 times a week. 2MD was administered through diet based on 5g daily diet consumption per mouse. Water was given *ad libitum.* Blood was taken via retro-orbital bleeding method under isoflurane anesthetic. Serum was isolated from blood samples, frozen and subsequently analyzed with a benchtop clinical chemistry analyzer (Pentra 400, Horiba ABX). Organ pictures were taken with a Canon EOS 5D DSLR with 24-105mm lens in RAW format.

5/6ths Nephrectomy Rat Model. Disease Induction. Weanling, male Sprague-Dawley rats were obtained from Harlan (Madison, WI). Following a 10-13 day acclimation period, the animals had two-thirds of one kidney removed. After a week, the other entire kidney was removed. The animals were then switched from a chow diet to a purified rodent diet (Suda et al., Purified Rodent Diet-Diet 11) containing 0.6% Ca and 0.9% phosphorus and fat soluble vitamins A, D, E and K. Water and diet were provided *ad libitum.*

Animal Husbandry. Animals were housed in suspended, plastic shoe-box style cages with corn cob bedding (prior to surgery) or in stainless steel, wire-bottom cages (approximately one week after surgery). The animal rooms were maintained at a temperature of 68 to 72°F and a relative humidity of 25 to 75%. The holding rooms were set to provide 12 hours of light per day.

Treatment Groups. Approximately four weeks after the second surgery, animals were assigned to treatment groups (14-15 animals/group) so that each group had the same average PTH level.

Dose Preparation (Vehicle Formulation). The negative control material was prepared by volumetrically measuring ethanol (5%) and Neobee oil, mixing and then placing in storage at 2 to 8 °C.

Dose Preparation (2MD Formulation). 2MD formulations (DP001, Sigma Aldrich Fine Chemicals, Madison, WI) were prepared by first determining the concentration of an ethanol stock solution using UV spectrophotometry (extinction coefficient = 42,000; □ₘₐₓ = 252 nm). The solutions were then volumetrically added to Neobee oil so that there was no more than 5% ethanol in the final solution. If necessary, additional ethanol was added to bring the final ethanol amount to 5%. The solution was mixed and stored at 2 to 8°C.

Dose Administration Method. Both vehicle and 2MD were administered orally to the back of the tongue at 0.5 ml/kg body weight once daily for 8 weeks.

Serum Parathyroid Hormone (PTH) Levels. By "serum PTH levels" we mean the amount of PTH released by the parathyroid gland. PTH is the most important regulator of the body's calcium and phosphorus levels, and is controlled by the level of calcium in the blood. Low blood calcium levels cause increased PTH to be released, while high blood calcium levels inhibit PTH release. Normal values are 10-55 picograms per milliliter (pg/mL). Four weeks after surgery and 4 and 8 weeks after treatment initiation, blood was collected from the tail artery and the concentration of bioactive serum PTH was measured using the rat BioActive Intact PTH ELISA Kit from Immutopics, Inc. (San Clemente, CA).

Serum Calcium Analysis. Four weeks following surgery and 4 and 8 weeks after treatment started, blood was collected from the tail artery of each experimental animal. The blood was allowed to coagulate at room temperature and then centrifuged at 3000 x g for 15 minutes. The serum was transferred to a polypropylene tube and stored frozen at -20°C. The level of calcium was determined by diluting the serum into 0.1% lanthum chloride and measuring the absorbance on an atomic absorption spectrophotometer (Perkin Elmer Model 3110, Shelton, CT).

Phosphorus Assay. Four weeks after surgery and 8 weeks after treatment started, blood was collected from the tail artery of each experimental animal. The blood was allowed to coagulate at room temperature and then centrifuged at 3000 x g for 15 minutes. The serum was transferred to a polypropylene tube and stored frozen at -20°C. The level of phosphorus was determined using a clinical analyzer (Pentra 400, Horiba ABX Diagnostics - France; UV method using phosphomolybdate).

Creatinine Assay. Measuring serum creatinine levels is a useful and inexpensive method of evaluating renal dysfunction. Creatinine is a non-protein waste product of creatinine phosphate metabolism by skeletal muscle tissue. Creatinine production is continuous and is proportional to muscle mass. Creatinine is freely filtered and therefore the serum creatinine level depends on the Glomerular Filtration Rate (GFR). Renal dysfunction diminishes the ability to filter creatinine and the serum creatinine rises. If the serum creatinine level doubles, the GFR is considered to have been halved. A threefold increase is considered to reflect a 75% loss of kidney function.

In the following examples, serum creatinine levels were evaluated four weeks after surgery and 8 weeks after treatment started. Blood was collected from the tail artery of each experimental animal. The blood was allowed to coagulate at room temperature and then centrifuged at 3000 x g for 15 minutes. The serum was transferred to a polypropylene tube and stored frozen at -20°C. The level of creatinine was determined using a clinical analyzer (Pentra 400, Horiba ABX Diagnostics - France; Jaffe reaction) are indicative of impaired renal function and chronic nephritis. In one embodiment of the invention, a decrease in serum creatinine levels of approximately 30% is expected after treatment according to the method of the present invention.

Proteinuria measurements. Proteinuria measurements were performed by putting gentle pressure on the bladder for spot urine analysis on a clean surface with a semi-quantitative dipstick (Combistix, Bayer). Mice were determined to have proteinuria when urinary protein output was 100 mg/dL or more. Urine was collected by placing the animals in metabolic cages and performing two-24 hr collections. Total protein was measured using a Bradford assay. (MM Bradford, Analytical Biochemistry 72: 248-254, 1976).

Histology. Half of a kidney was preserved in cold zinc formalin fixative, embedded in paraffin, and sectioned 4 µm thick. Hematoxylin and eosin (H&E) and Masson-Goldner's trichrome were used to study histological differences between kidney samples. Von Kossa staining was performed on whole heart sections (processed as described above) to determine heart calcification. Sectioning and staining was performed by the University of Wisconsin - Madison McArdle Histology Laboratory. Micrographs in the figures were taken by an Olympus BH-2 microscope and Nikon Coolpix 990 digital camera.

### Example 2: NOD Mouse Model of Type I Diabetic Nephropathy

Disease Induction. NOD mice (breeder pairs) were obtained from JAX laboratories (Bar Harbor, ME). Weanling mice were placed on chow diet (5K52 Lab Diets) or purified diet (Suda et al., Purified Rodent Diet-Diet 11) and allowed to spontaneously develop diabetes (two consecutive measurements of plasma glucose >250 mg). Water and diet were provided *ad libitum.*

Animal Husbandry. Animals were housed in autoclaved, plastic shoe-box style cages with corn cob bedding in groups of 2-4 animals/cage. Once mice were confirmed diabetic, the cages were changed daily. The animal rooms were maintained at a temperature of 68 to 72°F and a relative humidity of 25 to 75%. The holding rooms were set to provide 12 hours of light per day.

Treatment Groups. Mice were tested for elevated urinary glucose (>2000 mg; dipstick) two times per week. If the urine test was positive, a fasting blood glucose was performed the following day (spectrophotometer or glucometer). If positive, a second blood glucose test was done for confirmation. Once animals were determined to be diabetic, animals were assigned to treatment groups (6-12/group) so that each group would have the same average plasma glucose level at enrollment. The treatments were provided orally in the diet.

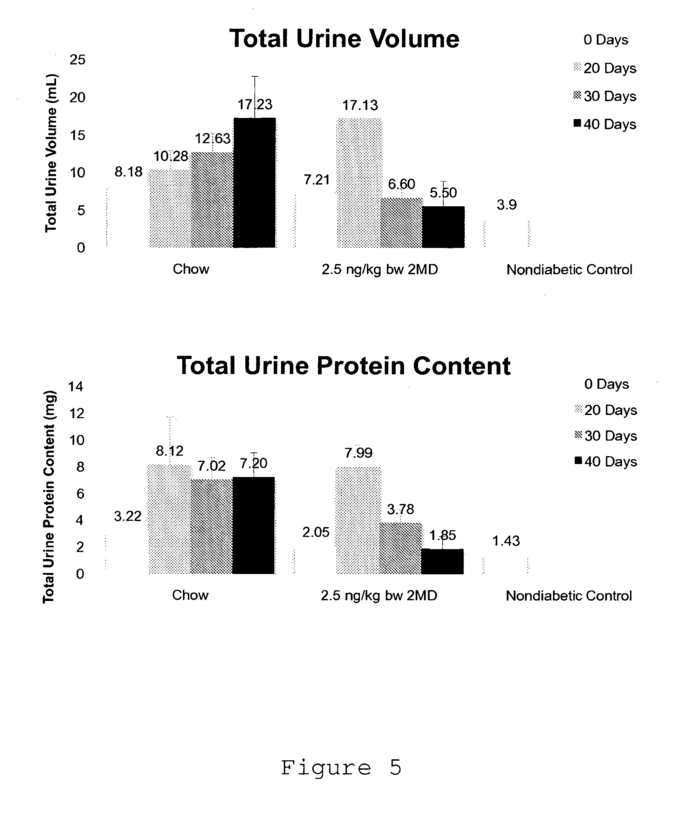

Diet Preparation. The negative control diet was prepared by volumetrically measuring ethanol (0.1% of the diet) and soybean oil (2% of the diet), vortexing and then adding to one half of the diet, mixing for 20 minutes before adding the remainder of the diet. The diet containing 2MD was prepared by first determining the concentration of a ethanol stock solution using UV spectrophotometry (extinction coefficient = 42,000; □ₘₐₓ = 252 nm). The solution was then volumetrically added to soybean oil so that there was no more than 0.1 % ethanol in the final solution. If necessary, additional ethanol was added to bring the final ethanol amount to 0.1 %. The ethanol and oil solution was vortexed, added to one-half the diet, mixed for 20 minutes and then the remaining diet added and mixed for another 20 minutes (Figure 5).

### Example 3: Proteinuria development is prevented by 2MD.

In this example, the inventors show the effect of 2MD on preventing NOD mice from developing proteinuria.

We selected doses of 2MD that would either cause: 1) significant hypercalemia (∼11 to 12 mg/dL or Δ∼1.5 to 2.5 mg/dL over control group); 2) mildly (∼10 to 11 mg/dL or Δ∼0.7 to 1.5 mg/dL over control group) increased serum calcium levels; or 3) negligible increase in serum calcium levels (∼10 mg/dL or Δ0.5 mg/dL over control group; Figure 6A). 2MD was effective in preventing proteinuria in a dose dependent manner, with the 5 ng 2MD/mouse/day dose completely preventing proteinuria (comparable to a 300 ng dose of 1,25(OH)₂D₃; Figure 6B). In addition to preventing proteinuria, 1 ng 2MD treatment delayed mean time of onset of proteinuria (Figure 6C). Growth of the mice was adversely affected with 5 ng 2MD treatment whereas a slight impact on growth was observed with 0.5 and 1 ng 2MD treatment (Figure 6D). The 5 ng 2MD treatment group had symptoms of hypercalcemia including unkempt fur and nervousness, but no lethargy or morbidity was observed in any 2MD dose groups.

In treatment of MLR/lpr mice with 1,25(OH)₂D₃ or 2MD where complete prevention of proteinuria was observed, there is a significant difference in the level of hypercalcemia. 300 ng 1,25(OH)₂D₃ elevated serum calcium levels to a sustained ∼13 mg/dL, whereas 5 ng 2MD only increases serum calcium to 12.4 mg/dL at the highest measured point, and 10.9 mg/dL at study termination (Figure 6A). This evidence suggests that the prevention of proteinuria (and amelioration of LN) is largely hormone (or analog) mediated. This hypothesis is reinforced when comparing the calcemic effects of 150 ng 1,25(OH)₂D₃, which elevate serum calcium levels to ∼12 mg/dL, to 1 ng 2MD, which serum calcium levels are within physiologically normal range throughout the study. Both show comparable efficacy in reducing proteinuria incidence (12-25% incidence), but hypercalcemia and its associated effects including weight loss are significantly less in the 1 ng 2MD group.

### Example 4: Glomerular damage is prevented by 2MD.

In this example, the inventors show that glomerular damage is prevented by administering therapeutically effective amounts of 2MD.

Kidneys from the control and 5 ng 2MD groups were harvested and stained for histological analysis. The most striking feature was the glomerular damage observed in the controls (Figure 7A and B) that was prevented with 2MD treatment (Figure 7E and F). Control kidneys exhibited glomerular sclerosis, fibrosis, basement membrane thickening and mesangial proliferation (Figure 7B). The 2MD treated kidneys had very little to no evidence of these features (Figure 7E). There was also a noticeable decrease in tubulointerstitial infiltration and a slight decrease in perivascular infiltration with treatment. Interstitial fibrosis was present in both control and 2MD kidneys (Figure 7C and F), but the extent of fibrotic staining was significantly less in the treatment group. Possibly the most encouraging finding was the lack of evidence of calcification in kidneys and hearts from mice treated with 2MD.

There was a clear change in renal histology between the control and 5 ng 2MD treatment groups. Glomerular damage is a primary factor in LN, as several downstream inflammatory events can be instigated by glomerular damage. Mesangial chemokine excretion and protein overload stimulation of proximal tubules can exacerbate renal damage caused by glomerular immune complex deposition and ensuing inflammation. By preventing the destruction of glomeruli, a key factor in LN pathogenesis, normal renal function is preserved and proteinuria is prevented. Interstitial infiltration of leukocytes was substantially less with 2MD treatment, and perivascular infiltration was also noticeably decreased. This could be from the reduced chemokine signaling that can be traced back to the prevention of glomerular damage. The reduced cellular infiltration is different from what we previously observed. This can be attributed to the particular strain of mice used in this experiment, the MRL/MpJ-Fas^{lpr}/J strain, which was recovered from cryopreservation because the MRL/MpJ-Fas^{lpr}/2J strain was progressively losing its ability to generate rapid and aggressive disease.

The most significant histological finding in terms of developing an LN therapeutic was the absence of calcification artifacts in kidneys and hearts from 2MD treated mice. 300 ng of 1,25(OH)₂D₃ was equally effective in preventing proteinuria as 5 ng of 2MD, but the 1,25(OH)₂D₃ treated group kidneys showed calcium crystals within renal structures (data not shown). The increased potency of 2MD combined with the decreased side effect of hypercalcemia and associated damage makes 2MD a promising candidate for development for LN treatment.

### Example 5: Splenomegaly is decreased with 2MD treatment.

In this example, the inventors show that splenomegaly is decreased with treatment according to the methods of the presnt invention.

5 ng 2MD significantly reduced splenomegaly compared to the 0 ng control group. Even with the decreased body weight compared to their control counterparts, 5 ng 2MD mice had smaller spleens, both visually (Figure 8A) and when measured for mass (Figure 8B) even when normalized to body weight (Figure 8C). Unlike the dose-dependent effect of 2MD observed with proteinuria incidence, reduction in splenomegaly was only observed in the 5 ng 2MD treatment group, which is associated with significant hypercalcemia. Histology revealed that splenomegaly in MRL/lpr mice causes disorganized white pulp structure (Figure 8E), whereas 5 ng 2MD treatment helps retain the sheath-like structure normally observed in non-diseased spleens.

In both the 300 ng 1,25(OH)₂D₃ and 2MD studies, only the highest dose groups which had the most severe hypercalcemia had a statistically significant reduction in spleen mass normalized to body weight. In addition, the 300 ng 1,25(OH)₂D₃ group, which produced higher serum calcium levels than the 5 ng 2MD group, also produced a greater reduction in spleen mass, and a better retention of normal spleen histology than observed with 5 ng 2MD treatment (Figure 8E). The 150 ng per day 1,25(OH)₂D₃ dose reduced spleen mass comparable to that achieved with 5 ng 2MD treatment. These results suggest that elevated serum calcium levels may play an important role in the reduction of splenomegaly in MRL/lpr mice.

## Claims

1. A therapeutically effective amount of a compound that is 2-methylene-19-nor-20(S)-1,25-dihydroxyvitamin D₃ or pharmaceutically acceptable salts thereof for use in preventing renal disease in a subject at risk of developing renal disease without inducing hypercalcemia in the subject.

2. The compound for use according to claim 1, wherein the renal disease is Lupus nephritis.

3. The compound for use according to claim 1, wherein the 2-methylene-19-nor-20(S)-1,25-dihydroxyvitamin D₃ is formulated in an oral, topical, transdermal, parenteral, injection or infusion dosage form.

4. The compound for use according to claim 1, wherein the therapeutically effective amount ranges from 0.001 µg/day to 5 µg/day per kg bodyweight.

5. A therapeutically effective amount of a compound that is 2-methylene-19-nor-20(S)-1,25-dihydroxyvitamin D₃ or pharmaceutically acceptable salts thereof for use in preventing symptoms of renal disease in a subject at risk of developing symptoms of renal disease without inducing hypercalcemia in the subject.

6. The compound for use according to claim 5, wherein the renal disease is Lupus nephritis.

7. The compound for use according to claim 5, wherein the symptoms of renal disease are selected from the group consisting of proteinuria, glomerular damage and splenomegaly.

8. A therapeutically effective amount of a compound that is 2-methylene-19-nor-(20s)-1α25 dihydroxy-vitamin D₃ (2MD) or pharmaceutically acceptable salts thereof for use in treating symptoms of renal disease in a subject having symptoms of renal disease without inducing hypercalcemia in the subject.

9. The compound for use according to claim 8, wherein the renal disease is Lupus nephritis.

10. The compound for use according to claim 8, wherein the symptoms of renal disease are selected from the group consisting of proteinuria and splenomegaly.

11. The compound for use according to claim 8, wherein the therapeutically effective amount of 2MD ranges from 0.001 µg/day to 5 µg/day per kg bodyweight.

12. The compound for use according to claim 11, wherein the therapeutically effective amount of 2MD ranges from 0.01 µg/day to 1 µg/day per kg bodyweight.

13. The compound for use according to claim 11, wherein the therapeutically effective amount of 2MD ranges from about 0.025 µg/day to about 0.3 µg/day per kg bodyweight.

## Patentansprüche

1. Therapeutisch wirksame Menge einer Verbindung, bei der es sich um 2-Methylen-19-nor-20(S)-1,25-dihydroxyvitamin D3 oder um pharmazeutisch verträgliche Salze davon handelt, zur Verwendung bei der Prophylaxe einer Nierenerkrankung bei einem Subjekt, bei dem die Gefahr der Entwicklung einer Nierenerkrankung besteht, ohne dass beim Subjekt Hyperkalzämie induziert wird.

2. Verbindung zur Verwendung nach Anspruch 1, wobei es sich bei der Nierenerkrankung um Lupus-Nephritis handelt.

3. Verbindung zur Verwendung nach Anspruch 1, wobei das 2-Methylen-19-nor-20(S)-1,25-dihydroxyvitamin D3 zu einer oralen, topischen, transdermalen, parenteralen, Injektions- oder Infusions-Dosierungsform zubereitet ist.

4. Verbindung zur Verwendung nach Anspruch 1, wobei die therapeutisch wirksame Menge im Bereich von 0,001 µg/Tag bis 5 µg/Tag pro kg Körpergewicht liegt.

5. Therapeutisch wirksame Menge einer Verbindung, bei der es sich um 2-Methylen-19-nor-20(S)-1,25-dihydroxyvitamin D3 oder um pharmazeutisch verträgliche Salze davon handelt, zur Verwendung bei der Prophylaxe von Symptomen einer Nierenerkrankung bei einem Subjekt, bei dem die Gefahr der Entwicklung von Symptomen einer Nierenerkrankung besteht, ohne dass beim Subjekt Hyperkalzämie induziert wird.

6. Verbindung zur Verwendung nach Anspruch 5, wobei es sich bei der Nierenerkrankung um Lupus-Nephritis handelt.

7. Verbindung zur Verwendung nach Anspruch 5, wobei die Symptome einer Nierenerkrankung aus der Gruppe ausgewählt sind, die aus Proteinurie, glomerulären Schädigungen und Splenomegalie besteht.

8. Therapeutisch wirksame Menge einer Verbindung, bei der es sich um 2-Methylen-19-nor-20(S)-1α,25-dihydroxyvitamin D3 (2MD) oder um pharmazeutisch verträgliche Salze davon handelt, zur Verwendung bei der Therapie von Symptomen einer Nierenerkrankung bei einem Subjekt, bei dem Symptome einer Nierenerkrankung auftreten, ohne dass beim Subjekt Hyperkalzämie induziert wird.

9. Verbindung zur Verwendung nach Anspruch 8, wobei es sich bei der Nierenerkrankung um Lupus-Nephritis handelt.

10. Verbindung zur Verwendung nach Anspruch 8, wobei die Symptome einer Nierenerkrankung aus der Gruppe ausgewählt sind, die aus Proteinurie und Splenomegalie besteht.

11. Verbindung zur Verwendung nach Anspruch 8, wobei die therapeutisch wirksame Menge von 2MD im Bereich von 0,001 µg/Tag bis 5 µg/Tag pro kg Körpergewicht liegt.

12. Verbindung zur Verwendung nach Anspruch 11, wobei die therapeutisch wirksame Menge von 2MD im Bereich von 0,01 µg/Tag bis 1 µg/Tag pro kg Körpergewicht liegt.

13. Verbindung zur Verwendung nach Anspruch 11, wobei die therapeutisch wirksame Menge von 2MD im Bereich von etwa 0,025 µg/Tag bis etwa 0,3 µg/Tag pro kg Körpergewicht liegt.

## Revendications

1. En quantité à effet thérapeutique, un composé qui est la 2-méthylidène-19-nor-20(S)-1,25-dihydroxy-vitamine D₃, ou l'un de ses sels pharmacologiquement admissibles, pour utilisation dans le but de prévenir une maladie rénale chez un sujet risquant de développer une maladie rénale, sans provoquer une hypercalcémie chez le sujet.

2. Composé pour utilisation conforme à la revendication 1, dans laquelle la maladie rénale est une néphrite lupique.

3. Composé pour utilisation conforme à la revendication 1 ou 2, pour laquelle la 2-méthylidène-19-nor-20(S)-1,25-dihydroxy-vitamine D₃ est formulée en une forme posologique pour voie orale, topique, transdermique ou parentérale, ou pour injection ou perfusion.

4. Composé pour utilisation conforme à la revendication 1, duquel la quantité à effet thérapeutique vaut de 0,001 µg/jour à 5 µg/jour, par kilogramme de poids corporel.

5. En quantité à effet thérapeutique, un composé qui est la 2-méthylidène-19-nor-20(S)-1,25-dihydroxy-vitamine D₃, ou l'un de ses sels pharmacologiquement admissibles, pour utilisation dans le but de prévenir des symptômes d'une maladie rénale chez un sujet risquant de développer des symptômes d'une maladie rénale, sans provoquer une hypercalcémie chez le sujet.

6. Composé pour utilisation conforme à la revendication 5, dans laquelle la maladie rénale est une néphrite lupique.

7. Composé pour utilisation conforme à la revendication 5, dans laquelle les symptômes de la maladie rénale sont choisis dans l'ensemble formé par une protéinurie, une lésion glomérulaire et une splénomégalie.

8. En quantité à effet thérapeutique, un composé qui est la 2-méthylidène-19-nor-20(S)-1α,25-dihydroxy-vitamine D₃ (2MD), ou l'un de ses sels pharmacologiquement admissibles, pour utilisation dans le but de traiter des symptômes d'une maladie rénale chez un sujet présentant des symptômes d'une maladie rénale, sans provoquer une hypercalcémie chez le sujet.

9. Composé pour utilisation conforme à la revendication 8, dans laquelle la maladie rénale est une néphrite lupique.

10. Composé pour utilisation conforme à la revendication 8, dans laquelle les symptômes de la maladie rénale sont choisis dans l'ensemble formé par une protéinurie et une splénomégalie.

11. Composé pour utilisation conforme à la revendication 8, pour laquelle la quantité à effet thérapeutique de 2MD vaut de 0,001 µg/jour à 5 µg/jour, par kilogramme de poids corporel.

12. Composé pour utilisation conforme à la revendication 11, pour laquelle la quantité à effet thérapeutique de 2MD vaut de 0,01 µg/jour à 1 µg/jour, par kilogramme de poids corporel.

13. Composé pour utilisation conforme à la revendication 11, pour laquelle la quantité à effet thérapeutique de 2MD vaut d'environ 0,025 µg/jour à environ 0,3 µg/jour, par kilogramme de poids corporel.
